# EUROPEAN PATENT SPECIFICATION

(11) **EP 1 884 238 B1**
(45) Date of publication and mention of the grant of the patent: **26.06.2013**
(21) Application number: 06729236.7
(22) Date of filing: 16.03.2006
(51) Int. Cl.: A61K 31/336, A61K 35/56, A61K 36/02, A61P 3/04, C07D 303/14

(54) **Fucoxanthinol as anti-obesity agent**
Fucoxanthinol als Mittel gegen Fettleibigkeit
Fucoxanthinol comme agent anti-obesite

(30) Priority: 24.05.2005 JP 2005150490
(43) Date of publication of application: 06.02.2008
(73) Proprietor: KANEKA CORPORATION, Osaka, (JP)
(72) Inventor: MIYASHITA, Kazuo, 3-1-1, Minato-cho, Hakodate-shi, Hokkaido 041-8611 (JP); HOSOKAWA, Masashi, 3-1-1, Minato-cho, Hakodate-shi, Hokkaido 041-8611 (JP); SASHIMA, Tokutake, 10 chome,Kita-ku,Sapporo-shi,Hokkaido 001-0021, (JP)
(74) Representative: TBK
(86) International application number: PCT/JP2006/305239
(87) International publication number: WO 2006/126325

(56) References cited:
- US-A1- 2005 014 219
- ASAI A ET AL: "Biotransformation of fucoxanthinol into amarouciaxanthin A in mice and HepG2 cells: formation and cytotoxiciy of fucoxanthin metabolites" DRUG METABOLISM AND DISPOSITION, WILLIAMS AND WILKINS., BALTIMORE, MD, US, vol. 32, no. 2, 1 January 2004 (2004-01-01), pages 205-211, XP003013406 ISSN: 0090-9556
- SUGAWARA T ET AL: "Brown algae fucoxanthin is hydrolyzed to fucoxanthinol during absorption by Caco-2 human intestinal cells and mice" JOURNAL OF NUTRITION, WISTAR INSTITUTE OF ANATOMY AND BIOLOGY, PHILADELPHIA, PA, US, vol. 132, no. 5, 1 January 2002 (2002-01-01), pages 946-951, XP003013407 ISSN: 0022-3166
- MAEDA HAYATO ET AL: "Fucoxanthin and its metabolite, fucoxanthinol, suppress adipocyte differentiation in 3T3-L1 cells." INTERNATIONAL JOURNAL OF MOLECULAR MEDICINE JUL 2006, vol. 18, no. 1, July 2006 (2006-07), pages 147-152, XP002479345 ISSN: 1107-3756
- MAEDA H. ET AL: 'Fuxocanthin from edible seaweed, Undaria pinnatifida, shows antiobesity effect through UCP1 expression in white adipose tissues' BIOCHEMICAL AND BIOPHYSICAL RESEARCH COMMUNICATIONS vol. 332, 06 May 2005, pages 392 - 397, XP004902490
- HOSOKAWA M. ET AL: 'Kaiso Shishitsu no Shirarezaru Kenko Kino, Kasso Fucoxanthin ni Shibo Chikuseki Yokuse nado no Kino' KAGAKU TO SEIBUTSU vol. 43, no. 3, March 2005, pages 150 - 152, XP003006019
- KONISHI I. ET AL: 'Gan Saibo ni Apoptosis o Yudo suru Ascidian Carotenoid no Tanri to Kino Kaiseki' NIPPON SUISAN GAKKAI KOEN YOSISHU vol. 2005, April 2005, page 128, XP003006020
- MAEDA H ET AL: "Fucoxanthin from edible seaweed, Undaria pinnatifida, shows antiobesity effect through UCP1 expression in white adipose tissues", BIOCHEMICAL AND BIOPHYSICAL RESEARCH COMMUNICATIONS, ACADEMIC PRESS INC. ORLANDO, FL, US, vol. 332, no. 2, 1 July 2005 (2005-07-01), pages 392-397, XP004902490, ISSN: 0006-291X, DOI: 10.1016/J.BBRC.2005.05.002

## Description

### Technical Field

The present invention relates to an anti-obesity active agent containing fucoxanthinol which is one of carotenoids, in particular, fucoxanthinol extracted from ascidian (sea squirt) as an active ingredient for use in the treatment of obesity.

Fucoxanthinol is such a substance that a -OCOCH₃ group of fucoxanthin which is also one of carotenoids is substituted by a -OH group. Fucoxanthin is specifically contained in marine algae (seaweed), in particular, brown algae (Phaeophyceae) and has been reported to have strong anticancer activity (Non-Patent Document 1). According to Non-Patent Document 1, anticancer activity of fucoxanthin is closely related to enhancement of activity of intranuclear transcription factor (PPAR γ : proxisome proliferator activated receptor γ) in cancer cells, and fucoxanthin exhibits far stronger antiproliferative activity than that of troglytazone which is a pharmacological agent known as a ligand for PPAR γ. In this connection, when troglytazone and fucoxanthin are used in combination, proliferation of large intestine cancer cells are suppressed more strongly than in the cases where these are used alone.

Further, fucoxanthin has been reported to have strong anti-obesity activity (Non-Patent Document 2). According to Non-Patent Document 2, it has been revealed that fucoxanthin has differentiation suppressing activity on adipocytes (fat cells) and that reductions in body weight and WAT (white adipose tissue) in the animal experiment in which wakame seaweed (Undaria pinnatifida) oil was administered to animals are attributable to inhibition of differentiation into adipocytes by fucoxanthin and to combustion of fat due to formation of UCP 1 (uncoupling protein 1) for which PPARγ in WAT is responsible.

As described above, it has been revealed that fucoxanthin exhibits strong activities as an anticancer active agent and an anti-obesity active agent. Now, the discussion will be focused on physiologically functional food materials related to anti-obesity. Particularly in Japan, polyphenols such as catechin, fravanedienol and the like has already been put to practical use and commercialized to develop a market. Also in Europe and the United States, health-consciousness has strongly been increasing due to health impairment caused by obesity, and accordingly, development of physiologically functional food materials has become intensively pursued. However, since studies on physiologically functional food materials having anti-obesity activities are in the infancy, there is a good possibility that physiologically functional food materials which exhibit anti-obesity activities will be found out beyond marine algae (seaweeds). In addition, the activities thereof can be stronger than that of fucoxanthin. If an anti-obesity active agent can be extracted easily and inexpensively (economically) from abundant material resources, it is possible to realize further expansion of the market of physiologically functional food materials.

Patent Document 1 discloses an invention which relates to a method for purifying fucoxanthin, i.e., a method for obtaining high purity fucoxanthin from marine algae. Patent Document 2 discloses an invention which relates to an anti-oxidizing agent and an anti-oxidation method that use fucoxanthin, and in which attention is focused on anti-oxidation ability of fucoxanthin extracted from algae such as diatoms (Bacillariophyceae) or the like, and fucoxanthin is used in foods, cosmetic products and pharmaceutical products to obtain anti-oxidizing agents. However, both of Patent Documents 1 and 2 are silent with respect to fucoxanthinol.

The activity of an anti-oxidizing agent means as described below. A part of oxygen necessary in a body is converted into active oxygen having strong oxidizability after intake of the oxygen. The active oxygen damages cells and oxidizes fats (lipids) in a body into lipid peroxides. The lipid peroxides increase blood viscosity, i.e., impair blood flow, and can cause arterial sclerosis or hypertension. A substance which suppresses such action of active oxygen is referred to as an anti-oxidizing agent.

Non-Patent Document 1: "Fucoxanthin induces apoptosis and enhances the antiproliferative effect of the PPAR γ ligand, troglitazone, on colon cancer cells" by Masashi Hosokawa, Masashiro Kudo, Hayato Maeda, Hiroyuki Kohno, Takuji Tanaka, and Kazuo Miyashita; Biochimica et BiophysicaActa, 1675, pp. 113-119 (2004)
Non-Patent Document 2: "Anti-obesity Effect (by Administration) of Wakame Seaweed (Undaria pinnatifida) Oil" by Takaaki Konno, Hayato Maeda, Masashi Hosokawa, Kazuo Miyashita, Kei Funayama, and Takahisa Nakano; Main Points of Lectures at Academic Meeting of The Japanese Society of Fisheries Science in 2005, p. 130.
Patent Document 1: Japanese Unexamined Patent Publication No. 2004-75634
Patent Document 2: Japanese Unexamined Patent Publication No. Hei 7(1995)-224278
Asai, A et al., "Biotransformation of fucoxanthinol into amarouciaxanthin A in mice and HepG2 cells: formation and cytotoxiciy of fucoxanthin metabolites" DRUG METABOLISM AND DISPOSITION, WILLIAMS AND WILKINS., BALTIMORE, MD, US, vol. 32, no. 2, (2004), pages 205-211, discloses the biotransformation of fucoxanthin into the two metabolites fucoxanthinol and amarouciaxanthin A. This biotransformation takes place in the plasma after the oral administration of fucoxanthin in mice.
US 2005/014219 A describes the isolation of a carotengenic biosynthetic gene cluster from a strain of *Panteoa stewartii,* while the products of the genes of this gene cluster are useful for the conversion of farnesyl pyrophosphate to carotenoids. As an example of such a carotenoid, fucoxanthinol is explicitly mentioned.
Sugawara T, et al., "Brown algae fucoxanthin is hydrolyzed to fucoxanthinol during absorption by Caco-2 human intestinal cells and mice" JOURNAL OF NUTRITION, WISTAR INSTITUTE OF ANATOMY AND BIOLOGY, PHILADELPHIA, PA, US, vol. 132, no. 5, (2002), pages 946-951, describes the hydrolization of fucoxanthin originating from brown algae into fucoxanthinol during absorption by Caco-2 human intestinal cells and in mice.
Maeda H. et al.: "Fucoxanthin from edible seaweed, Undaria pinnatifida, shows antiobesity effect through UCP1 expression in white adipose tissues"; Biochemical and Biophysical Research Communications, Vol. 332, Pages 392-397 is directed to the use of fucoxanthin, which contributes to the reducing of abdominal white adipose tissue in rats and mice.

### Disclosure of Invention

### Problem to be Solved by the Invention

As described above, fucoxanthin is effective as an anti-obesity active agent. However, there has been a problem that fucoxanthin is contained in mature (adult) brown algae (Phaeophyceae) in a small amount, and accordingly, it takes a long time and high cost to obtain fcoxanthin in an amount sufficient for practical use. Further, a substance which shows a stronger effect as an anti-obesity active agent has been desired. It is, therefore, an object of the present invention to provide an anti-obesity active agent and an anti-obesity method which are capable of overcoming the drawback inherent in the conventional techniques and which use a carotenoid exhibiting an anti-obesity activity stronger than that of fucoxanthin.

### Means to Solve the Problem

The anti-obesity active agent of the present invention characteristically contains fucoxanthinol as an active ingredient. The present inventors have made intensive and extensive studies as to what carotenoid has an anti-obesity activity stronger than that of fucoxanthin and what material should be used in order to economically obtain such a carotenoid. As a result, they have found the answer that the carotenoid is fucoxanthinol. Based on many experiments, they have confirmed that the anti-obesity activity of fucoxanthinol is stronger than that of fucoxanthin. In consequence, use of fucoxanthinol in an amount which is the same as or even smaller than that of fucoxanthin enables an anti-obesity active agent having its anti-obesity effect greater than that of fucoxanthin to be realized.

Further, the anti-obesity active agent of the present invention is in the form of a food, fertilizer, or pharmaceutical product which contains fucoxantinol added thereto. As described above, fucoxanthinol per se is an anti-obesity active agent. By adding fucoxanthinol to a food, fertilizer, or pharmaceutical product, a physiologically functional food material, fertilizer, pharmaceutical product or the like which has an anti-obesity activity can be prepared. In this case, since strength of the anti-obesity activity of the product can be controlled by changing the amount of fucoxanthinol contained therein, the amount may appropriately be adjusted according to purpose. In the present invention, the food, fertilizer, or pharmaceutical product which contains fucoxanthinol added thereto is also referred to as a fucoxanthinol anti-obesity active agent.

In the anti-obesity active agent of the present invention, the fucoxanthinol is extracted preferably from an organism (a biological body). It is preferred that the organism (biological body) be sea squirt (ascidian). The term "sea squirt (ascidian) is a generic name of marine animals which belong to class Ascidiacea, subphylum Urochordata (Tunicata), phylum Chordata and which inhabit nationwide. As described above, sea squirt (ascidian) is abundantly found as resources. In the present invention, fucoxanthinol is extracted particularly from internal organs (visceral organs) and hard skins of sea squirt (ascidian). Accordingly, since meat of sea squirt is used as food and fucoxanthinol is extracted from the remaining hard skins and internal organs, it takes not so much time or costs not so much to obtain fucoxanthinol in an amount sufficient for practical use. This enables the problem inherent in the conventional techniques to be solved.

In addition, it has been revealed by the studies that fucoxanthinol extracted from sea squirt (ascidian) shows an anti-obesity activity stronger than that of fucoxanthin fractionally collected from wakame seaweed (Undaria pinnatifida). Therefore, finding out of a carotenoid exhibiting a stronger anti-obesity activity, which has heretofore been the challenge, has been achieved.

The anti-obesity active agent of the present invention preferably further contains fucoxanthin fractionally collected from wakame seaweed (Undaria pinnatifida) and added thereto. In other words, the anti-obesity active agent of the present invention may be one to which fucoxanthin that has already been used is added in addition to fucoxanthinol characteristically used in the present invention. By virtue of this formulation, anti-obesity activity of fucoxanthin and anti-obesity activity of fucoxanthinol are combined to exhibit further enhanced anti-obesity activity. In this connection, amounts of fucoxanthin and fucoxanthinol to be added may appropriately be adjusted.

The term "sea squirt (ascidian) is a generic name of marine animals which belong to class Ascidiacea, subphylum Urochordata (Tunicata), phylum Chordata and which abundantly live nationwide. In the present invention, fucoxanthinol is extracted particularly from internal organs and hard skins of sea squirt (ascidian). Accordingly, it takes not so much time or costs not so much to obtain fucoxanthinol in an amount sufficient for practical use. Further, it has been revealed by the studies that fucoxanthinol extracted from sea squirt (ascidian) shows anti-obesity effect stronger than that of fucoxanthin fractionally collected from wakame seaweed (Undaria pinnatifida). Moreover, by concomitantly using fucoxanthin fractionally collected from wakame seaweed (Undaria pinnatifida), the anti-obesity effect of fucoxanthin and the anti-obesity effect of fucoxanthinol combine to enable the anti-obesity method of the present invention to exhibit extremely enhanced effect.

### Effect of the Invention

It has been realized to extract fucoxanthinol from sea squirt (ascidian) which shows anti-obesity activity stronger than that of fucoxanthin extracted from wakame seaweed (Undaria pinnatifida) which has already been used. By virtue of this, it has become possible to obtain the anti-obesity active agent more inexpensively in a greater amount than in the past, and further, to provide the anti-obesity method which is cost-saving and extremely effective. By adding fucoxanthinol to a food, fertilizer, agricultural chemical, cosmetic product or pharmaceutical product, an anti-obesity physiologically functional food material, an anti-obesity fertilizer, an anti-obesity pharmaceutical product or the like can be prepared. Accordingly, it is expected to greatly contribute to expansion of the market of, in particular, physiologically functional foods.

### Best Mode for Carrying Out the Invention

In the following, fucoxanthinol used in the present invention and embodiment of the anti-obesity active agent using the same according to the present invention will be described with reference to the drawings. It should be noted, however, that the present invention is by no means restricted to the embodiment.

Now, description will be given on a method for extracting fucocanthinol used in the present invention. Such a method was employed that all of the carotenoids contained in sea squirt were extracted, followed by isolation. First, internal organs and hard skins of sea squirt (ascidian) were removed. The internal organs and the hard skins were subjected to extraction for 4 days using acetone in an amount twice the total weight of the internal organs and the hard skins. This procedure was repeated twice.

The acetone extract of sea squirt (ascidian) was introduced into a silica gel column, and neutral lipids, particularly triacylglycerol, were removed using a mixture of n-hexane and diethyl ether (volume ratio = 95 : 5), and then crude carotenoid fractions were eluted.

The crude carotenoid fractions were spotted on a silica gel thin-layer chromatography plate and developed using a mixture of acetone and n-hexane (volume ratio =4:6). Based on the spots assuming a yellow color or orange color as references, carotenoids were roughly divided into 5 fractions (Fractions 1 to 5) and eluted from the silica gel using acetone.

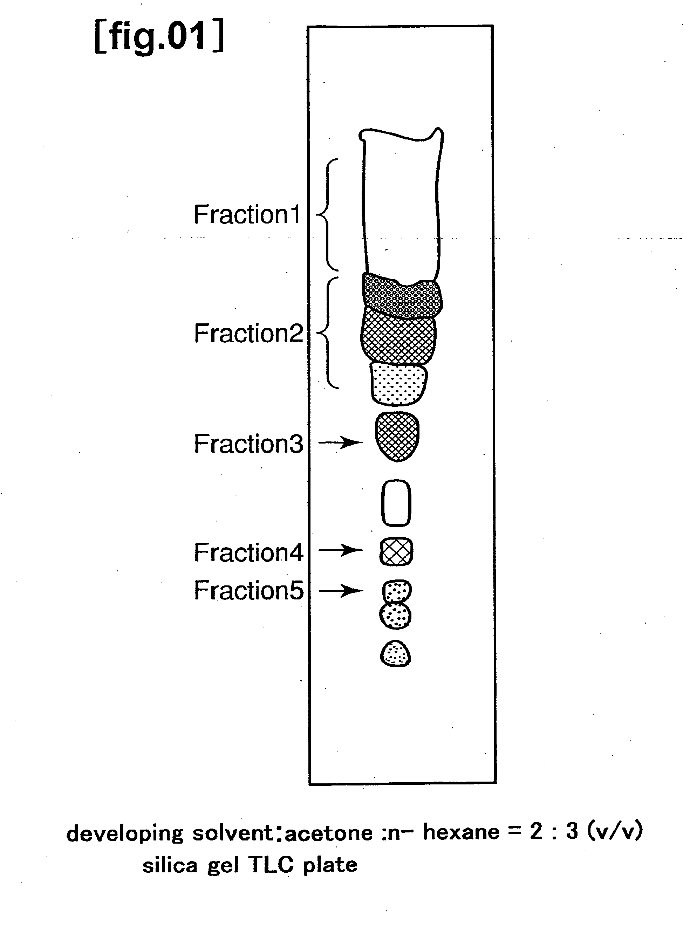

In Fig.1, a schematic representation of a thin-layer chromatogram of the sea squirt (ascidian) extract is shown. The thin-layer chromatogram was photographed, and the photographed chromatogram was schematically depicted (as Fig.1). Various carotenoids were extracted in Fractions 1 to 5. Specifically, halosinthiaxanthin and fucoxanthinol ware contained in Fraction 3 and Fraction 4, respectively. To carry out cell tests and structural analyses for the identifications, the following purifications were further performed.

Purification of Fraction 3: Fraction 3 was spotted on an RP-8 reversed-phase thin-layer chromatography plate (RP-8: a support for separation which is made of octyl silane) and developed using a mixture of methanol and water (volume ratio = 9 : 1), and then an orange fraction was separated and eluted with acetone.

Purification of Fraction 4 (fucoxanthinol): Fraction 4 was spotted on an RP-18 reversed-phase thin-layer chromatography plate (RP-18: a support for separation which is made of octadecyl silane) and developed using a mixture of methanol, acetone and water (volume ratio = 4 : 6 : 2), and then an orange fraction was separated and eluted with acetone.

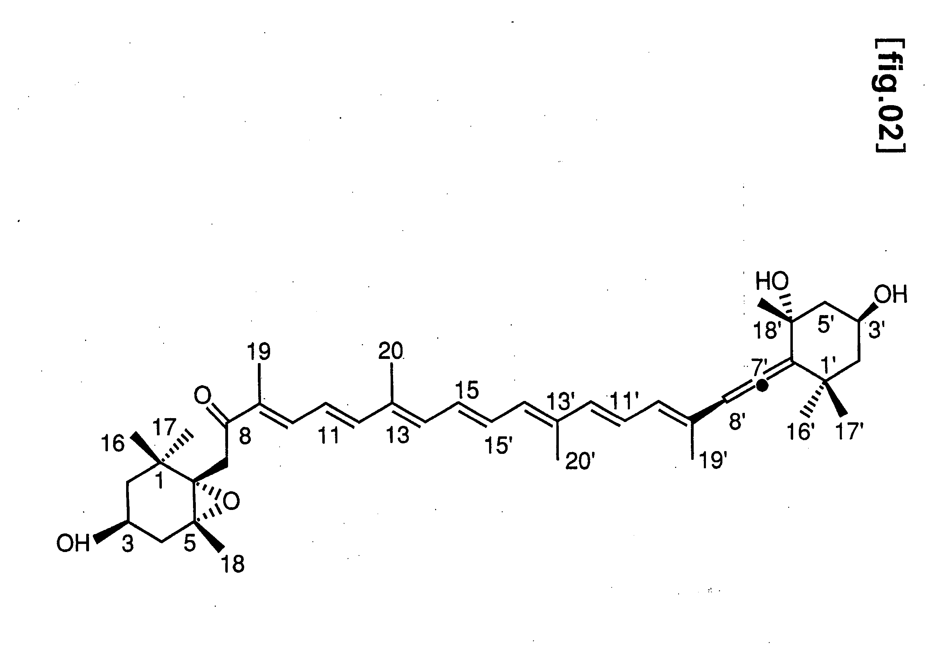

Structures of halosinthiaxanthin and fucoxanthinol were identified by mass spectrometric analysis and NMR analysis. Fig.2 shows the identified structure of fucoxanthinol.

Next, description will be given on anti-obesity activities of fucoxanthinol extracted from sea squirt (ascidian) and fucoxanthin. The experimental procedure was as follows.

Cell Culture: 3T3-L1 preadipocytes were incubated using DMRM culture medium containing 10% (by volume) of FBS at 37°C in the presence of CO₂ (5%). In this connection, as the FBS, one which had been inactivated at 56°C for 30 minutes was used. As the cells, those which were unused (naïve) and which had been kept in liquid nitrogen were used in each experiment. On a 96 well microplate, 5 x 10⁴ cells of the 3T3-L1 preadipocytes were seeded per well and pre-incubated for 48 hours.

Then, the culture medium was removed from the wells, and DMEM culture medium containing insulin (10 µg/ml), dexametazone (0.25 µg/ml) and IBMX (0.5 mM) was added to the wells, and incubation was further performed for 48 hours. Thereafter, differentiation to adipocytes was induce while repeating medium replacement at intervals of 4 days with the use of DMEM culture medium containing 5 µg/ml of insulin only. In this procedure, fucoxanthin was added to the culture medium to examine influence thereof on the differentiation induction.

Quantitative Determination of Intracellular Lipids with Oilred O: The cells incubated in the 96 well microplate were immobilized with formalin, and then an oilred O solution was added thereto to stain lipids in the cells. After the staining, the cells were washed with distilled water three times and observed. Subsequently, isopropanol was added to each of the wells to elute the pigment (colorant), and absorbance, i.e., optical density at 490 nm was measured using a microplate reader.

Measurement of GPDH Activity: For measurement of GPDH (glycerol-3-phosphate-dehydrogenase) activity, a GDPH activity measuring kit (manufactured by Hokudo Co., Ltd.) was used. Specifically, 3T3-L1 preadipocytes were pre-incubated on a 24 well microplate) for 48 hours, and then differentiation to adipocytes was inducedin the same manner as described above. After the incubation, the culture medium was removed, and the wells were washed with PBS, and then the enzyme extract was added to the wells to collect the cells. Subsequently, the cells were homogenized with an ultrasonic homogenizer and centrifuged at 12,800 g, and the supernatant was collected to measure enzyme activity.

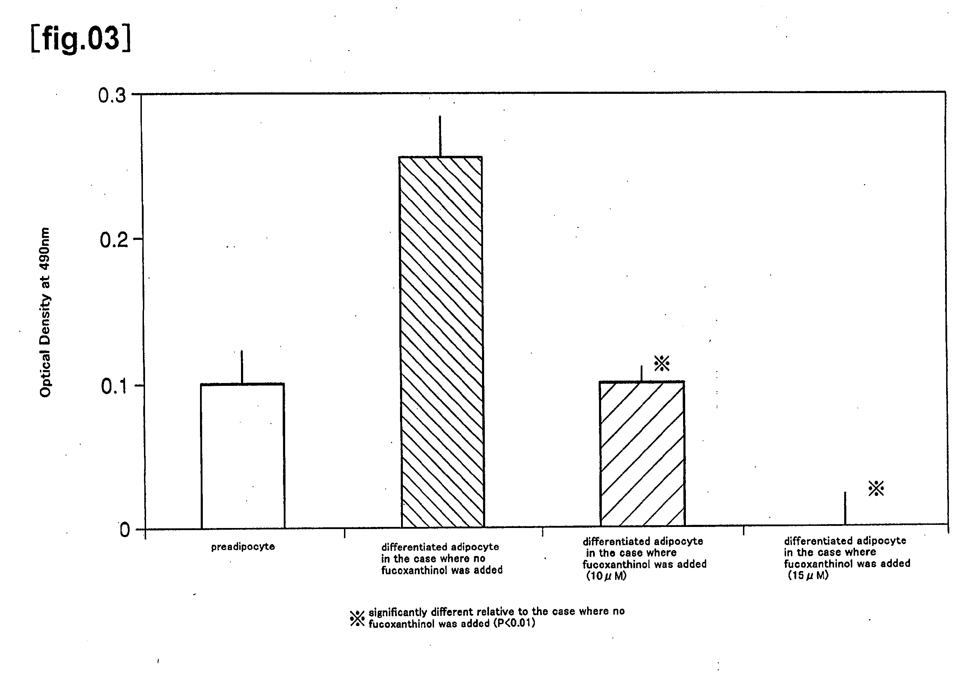

In the following, detailed description will be given on the results obtained with reference to Figs.3 to 5.

Fat (Lipid) Accumulation Controlling Effect: Fig.3 is an illustrative representation showing influence of fucoxanthinol on fat (lipid) accumulation associated with the differentiation of 3T3-L1 pre-adipocytes to 3T3-L1 adipocytes. The ordinate axis represents absorbance, i.e., optical density at 490nm which was measured using microplate reader in the quantitative determination of intracellular lipid with the use of Oilred O. In this connection, a larger value of the absorbance means a larger amount of adipocytes. As is apparent from Fig.3, due to the differentiation of preadipocytes (3T3-L1), fats (lipids) accumulated in the cells in a large amount. As opposed to this, it is seen that the fat (lipid) accumulation was markedly controlled by addition of fucoxanthinol. The controlling effect, which was enhanced in proportion to increase of the amount of the added fucoxanthinol, was clearly seen. This result shows anti-obesity activity of fucoxanthinol at the cell level. Therefore, the addition of fucoxanthinol means addition of an anti-obesity active agent.

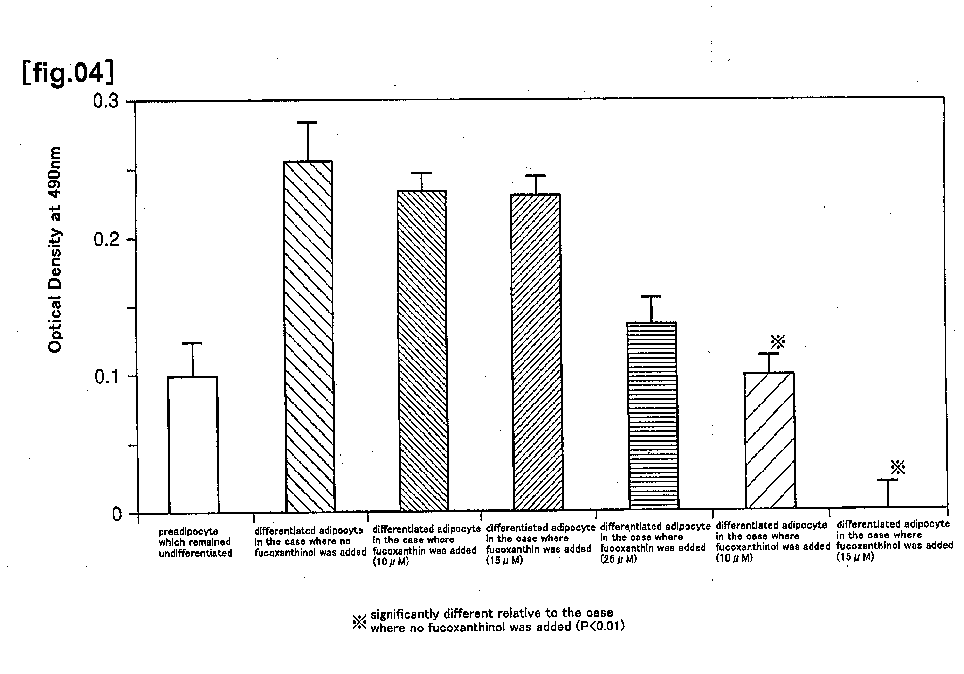

Fig.4 is an illustrative representation showing influences of fucoxanthin and fucoxanthinol on fat (lipid) accumulation associated with the differentiation of 3T3-L1 preadipocytes to 3T3-L1 adipocytes. Fig.4 is such a representation that activity of fucoxanthin fractionally collected from wakame seaweed (Undaria pinnatifida) is further incorporated in Fig.3. As is evident from Fig.4, the anti-obesity activity of fucoxanthin fractionally collected from wakame seaweed (Undaria pinnatifida) is apparently lower than that of fucoxanthinol. In this connection, when fucoxanthin fractionally collected form wakame seaweed (Undaria pinnatifida) and fucoxanthinol extracted from sea squirt (ascidian) are added in combination, the effects of them combine together (the results are not shown). Accordingly, further enhanced anti-obesity effec can be expected. Fucoxanthin fractionally collected from wakame seaweed (Undaria pinnatifida) may be added in addition to fucoxanthinol extracted from sea squirt (ascidian), according to need.

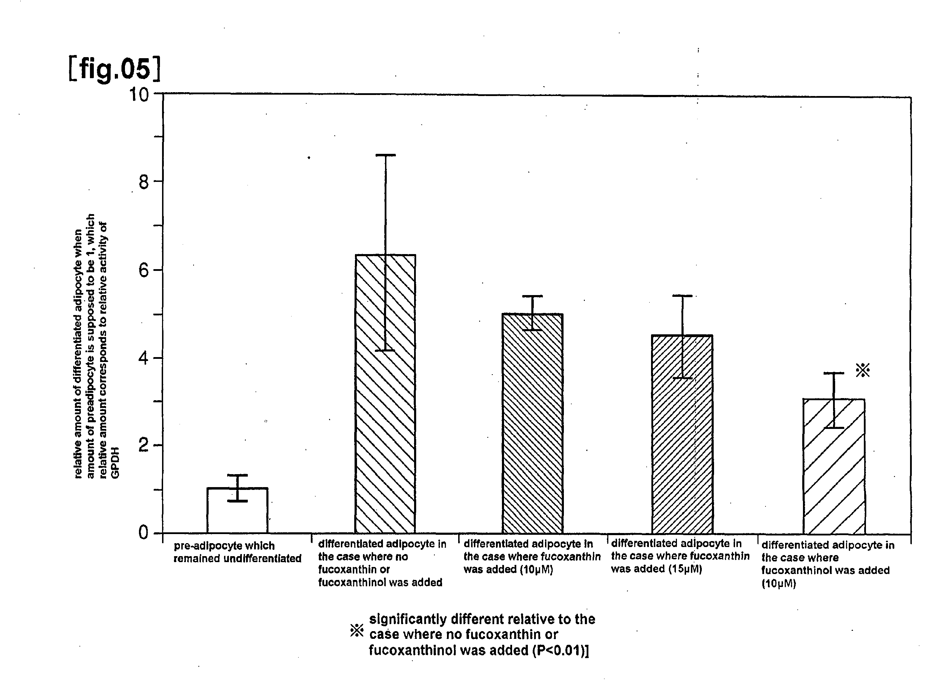

Fig.5 is an illustrative representation showing influencea of fucoxanthin and fucoxanthinol on fat (lipid) accumulation associated with the differentiation of 3T3-L1 preadipocytes to 3T3-L1 adipocytes. The ordinate axis represents relative amount of the adipocytes when amount of the preadipocytes is supposed to be 1. The relative amount corresponds to relative GPDH activity. In other words, it is also said that the ordinate axis represents relative GPDH activity. Therefore, a larger value of the amount means a higher GPDH activity. It is apparent from Fig.5 that activity of the enzyme (GPDH) which is responsible for fat (lipid) accumulation in cells was lowered by the addition of fucoxanthinol. This results show that at least a part of the fat (lipid) accumulation controlling effect of fucoxanthinol is attributable to inhibition of the lipogenic enzyme activity. On the other hand, no significant differences were observed between the cases where fucoxanthin fractionally collected from wakame seaweed (Undaria pinnatifida) was added and the case where neither fucoxanthinol nor fucoxanthin was added.

Then, description will be given on the results of animal experiments carried out.
Animals and Feed: For the animal experiments, KK-Ay mice (female; 3-week-old) were used. Feed for the experiments was prepared in accordance with AIN-93G composition (formulation). Amount of lipid in the feed was 13.5%. In the case where fucoxanthinol was added, 0.2% of the lipid in the feed was replaced with fucoxanthinol. After the preparation, the feed was immediately vacuum-packed and stored at -20°C or lower until feeding.

Breeding and Analysis: Preliminary Breeding was conducted for a week using the feed for the control group, and individuals which grew with no problem were divided into groups each consisting of 6 mice in such a manner that there were no substantial unevennesses in body weight among the groups. With respect to each of the groups, two mice were housed and bred in each plastic cage spread with sterilized wood chips to cover the floor. In the breeding cage, temperature and humidity were kept at 23±1°C and 50%, respectively, and each of light and dark periods was 12 hours. The mice were allowed to freely take (ingest) the feed and water, and the breeding with each of the feeds for the experiments was carried out for 24 days.

After termination of the feeding period, the mice were fasted for 12 hours. Thereafter, blood samples were collected from neck vessels in the presence of EDTA under anesthesia with (ethyl) ether. After the collection of blood samples, organs were immediately harvested and washed well with physiological saline to sufficiently clear blood therefrom. Livers were weighed and then stored at - 40°C or lower until subjected to analyses. Leptin concentrations and blood-sugar levels in blood were measured using a commercially available kit.

States of Animals in Breeding Period and after Termination of Breeding: With respect to increase in body weight and amount of feed intake, no significant differences were observed between the control group and the fucoxanthin-administered groups. Further, it was confirmed that they had no abnormalities in feed intake, glossiness of hair or action. Moreover, no significant differences were observed between both the groups in weight of liver, weight of small intestine or weight of hepatic lipid.

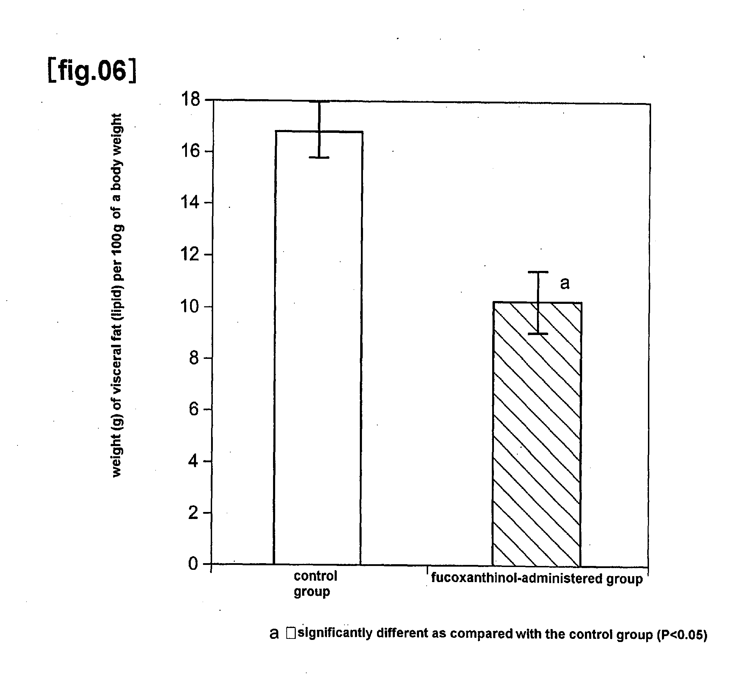

Visceral Fat Reducing Activity of Fucoxanthinol: Fig.6 shows comparison between the control group and the fucoxanthinol-administered group with respect to weight (g) of visceral fat per 100g of body weight. As shown in Fig.6, it is observed that weight of visceral fat was significantly reduced by the administration of fucoxanthinol. It was thereby found that fucoxanthinol has fat (lipid) reducing activity.

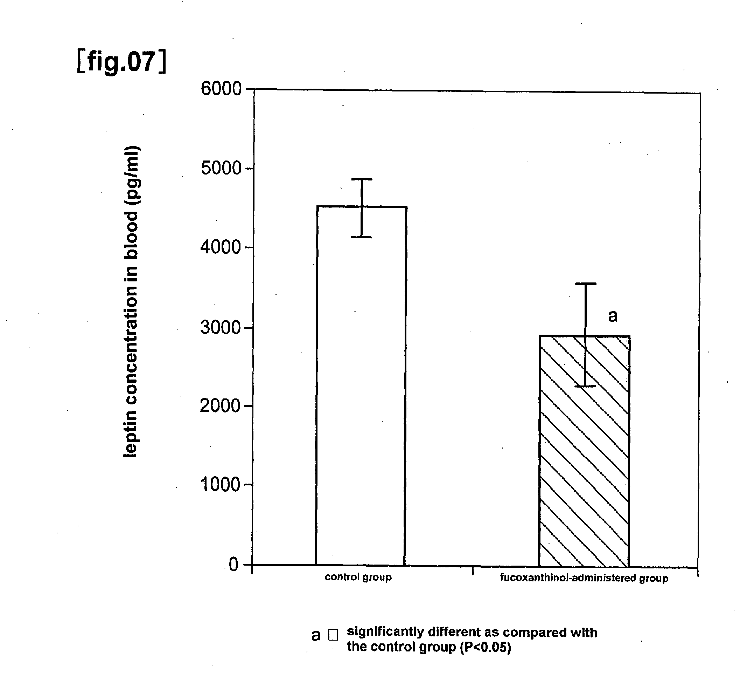

Leptin and Blood Sugar Levels Lowering Activity of Fucoxanthinol: Fig.7 shows comparison between the control group and the fucoxanthinol-administered group with respect to leptin concentration (pg/ml) in blood. The KK-Ay mice used in the experiments were those in obese and diabetic condition, and thus increase of visceral fat and increase of blood sugar level were caused. Due to the increase of visceral fat, the leptin concentration in blood was increased. However, by virtue of the administration of fucoxanthinol, it was observed that the leptin concentration was significantly reduced.

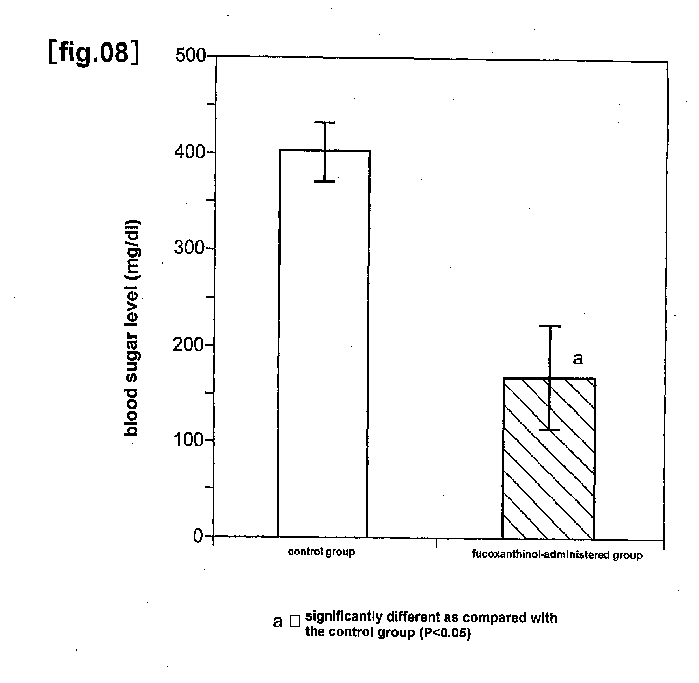

Fig.8 shows comparison between the control group and the fucoxanthinol-administered group with respect to blood sugar level (mg/dl). As is apparent from Fig.8, it was confirmed that also blood sugar level was reduced by the administration of fucoxanthinol. The result shows reducing effect of fucoxanthinol on the blood factors associated with obesity.

As described above, the present inventors have made researches and studies on carotenoids showing anti-obesity activity in various fields and materials. As a result, they have revealed that fucoxanthinol extracted from sea squirt (ascidian) has anti-obesity activity stronger than that of fucoxanthin fractionally collected from wakame seaweed (Undaria pinnatifida). It has thereby become possible to obtain an anti-obesity active agent more inexpensively and in a larger amount than ever before, enabling establishment of an effective anti-obesity treatment. The physiologically functional foods containing fucoxanthinol added thereto are expected to greatly contribute to expansion of the market of physiologically functional food materials.

### Industrial Applicability

The present invention provides an anti-obesity active agent containing fucoxanthinol as an active ingredient for use in the treatment of obesity. It is preferred that fucoxanthinol be extracted from sea squirt (ascidian). Foods to which fucoxanthinol is added serve as physiologically functional foods. Accordingly, in terms of industrial fields, the present invention can advantageously be used in the field of foods, particularly physiologically functional foods, and in addition thereto, the present invention is applicable in the fields of medicine and agriculture.

### Brief Description of Drawings

Fig.1 shows a thin-layer chromatogram of sea squirt extract in relation to fucoxanthinol used in the present invention.
Fig.2 shows a structural formula of fucoxanthinol used in the present invention.
Fig.3 is an illustrative representation showing influence of fucoxanthinol used in the present invention on fat (lipid) accumulation associated with the differentiation of 3T3-L1 preadipocytes to 3T3-L1 adipocytes.
Fig.4 is an illustrative representation showing influence of fucoxanthinol used in the present invention on fat (lipid) accumulation associated with the differentiation of 3T3-L1 preadipocytes to 3T3-L1 adipocytes. In this connection, this figure shows the influence in comparison with that of fucoxanthin.
Fig.5 is an illustrative representation showing influence of fucoxanthinol used in the present invention on increase in GPDH activity associated with the differentiation of 3T3-L1 preadipocytes to 3T3-L1 adipocytes. In this connection, this figure shows the influence in comparison with that of fucoxanthin.
Fig.6 is an illustrative representation showing influence of fucoxanthinol used in the present invention on weight of visceral fat (lipid) in the animal experiments.
Fig.7 is an illustrative representation showing influence of fucoxanthinol used in the present invention on leptin concentration in blood in the animal experiments.
Fig.8 is an illustrative representation showing influence of fucoxanthinol used in the present invention on blood sugar level in the animal experiments.

## Claims

1. An anti-obesity active agent containing fucoxanthinol for use in suppressing obesity.

2. The anti-obesity active agent of claim 1 for use according to claim 1, which is in the form of a pharmaceutical product.

3. The anti-obesity active agent of claim 1 or 2 for use according to claim 1 or 2,
wherein the fucoxanthinol is extracted from a biological body.

4. The anti-obesity active agent of claim 3 for use according to claim 3, wherein the biological body is sea squirt (ascidian).

5. The anti-obesity active agent of any of claims 1 to 4 for use according to any one of claims 1 to 4, which further contains fucoxanthin fractionally collected from wakame seaweed (Undaria pinnatifida) and added thereto.

## Patentansprüche

1. Wirkstoff gegen Fettleibigkeit, der Fucoxanthinol enthält, zur Verwendung beim Unterdrücken von Fettleibigkeit.

2. Wirkstoff gegen Fettleibigkeit nach Anspruch 1 zur Verwendung nach Anspruch 1, welcher in der Form eines pharmazeutischen Produkts vorliegt.

3. Wirkstoff gegen Fettleibigkeit nach Ansprüchen 1 oder 2 zur Verwendung nach Anspruch 1 oder 2, wobei das Fucoxanthinol aus einem biologischen Körper extrahiert ist.

4. Wirkstoff gegen Fettleibigkeit nach Anspruch 3 zur Verwendung nach Anspruch 3, wobei der biologische Körper eine Seescheide (Ascidiacea) ist.

5. Wirkstoff gegen Fettleibigkeit nach einem der Ansprüche 1 bis 4 zur Verwendung nach einem der Ansprüche 1 bis 4, welcher ferner Fucoxanthin enthält, das aus Wakame-Seegras (Undaria pinnatifida) fraktionell gesammelt und dazu zugegeben ist.

## Revendications

1. Agent à activité anti-obésité contenant du fucoxanthinol à utiliser pour se débarrasser de l'obésité.

2. Agent à activité anti-obésité de la revendication 1 à utiliser selon la revendication 1, qui est sous la forme d'un produit pharmaceutique.

3. Agent à activité anti-obésité de la revendication 1 ou 2, à utiliser selon la revendication 1 ou 2, dans lequel le fucoxanthinol est extrait d'un corps biologique.

4. Agent à activité anti-obésité de la revendication 3 à utiliser selon la revendication 3, dans lequel le corps biologique est le tunicier (ascidie).

5. Agent à activité anti-obésité de l'une des revendications 1 à 4 à utiliser selon l'une quelconque des revendications 1 à 4, qui contient en outre de la fucoxanthine collectée par fractionnement à partir de l'algue wakame (Undaria pinnatifida) et ajoutée à celui-ci.
